(19) **Europäisches Patentamt / European Patent Office / Office européen des brevets**

(11) **EP 2 275 113 B1**

(12) # EUROPEAN PATENT SPECIFICATION

(45) Date of publication and mention of the grant of the patent:
**25.07.2018 Bulletin 2018/30**

(21) Application number: **09725050.0**

(22) Date of filing: **26.03.2009**

(51) Int Cl.:
*A61K 36/18* (2006.01)  *A61K 8/49* (2006.01)
*A61K 8/97* (2017.01)  *A61K 31/215* (2006.01)
*A61K 31/365* (2006.01)  *A61K 36/00* (2006.01)
*A61K 36/73* (2006.01)  *A61Q 19/00* (2006.01)
*A61P 29/02* (2006.01)

(86) International application number:
**PCT/JP2009/001363**

(87) International publication number:
**WO 2009/119099 (01.10.2009 Gazette 2009/40)**

(54) **SKIN CIRCULATION-IMPROVING AGENT AND SKIN TEMPERATURE-ELEVATING AGENT**

MITTEL ZUR VERBESSERUNG DER HAUTZIRKULATION UND MITTEL ZUR ERHÖHUNG DER HAUTTEMPERATUR

AGENT AMÉLIORANT LA CIRCULATION CUTANÉE ET AGENT AUGMENTANT LA TEMPÉRATURE DE LA PEAU

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **28.03.2008 JP 2008085350**

(43) Date of publication of application:
**19.01.2011 Bulletin 2011/03**

(73) Proprietor: **Shiseido Company, Ltd.**
**Chuo-ku**
**Tokyo 104-8010 (JP)**

(72) Inventors:
• **MORI, Keiko**
**Yokohama-shi**
**Kanagawa 224-8558 (JP)**
• **KATSUYAMA, Masako**
**Yokohama-shi**
**Kanagawa 224-8558 (JP)**
• **GOZU, Yoko**
**Yokohama-shi**
**Kanagawa 224-8558 (JP)**
• **HAZE, Shinichiro**
**Yokohama-shi**
**Kanagawa 224-8558 (JP)**
• **JOUICHI, Atsushi**
**Yokohama-shi**
**Kanagawa 224-8558 (JP)**

• **TERAJIMA, Yushi**
**Yokohama-shi**
**Kanagawa 224-8558 (JP)**

(74) Representative: **Scholz, Volker et al**
**Boehmert & Boehmert**
**Anwaltspartnerschaft mbB**
**Pettenkoferstrasse 22**
**80336 München (DE)**

(56) References cited:
WO-A1-2005/023968  DE-A1-102005 006 791
JP-A- 2006 241 044  JP-A- 2006 290 802

• HONGRATANAWORAKIT T ET AL: "Evaluation of the harmonizing effect of Ylang -Ylang oil on humans after inhalation", MEDICINAL & AROMATIC PLANTS ABSTRACTS, SCIENTIFIC PUBLISHERS, NEW DELHI - INDIA, vol. 26, no. 5, 1 October 2004 (2004-10-01), XP018000746,
• DATABASE TCM [Online] SIPO; 20 June 2005 (2005-06-20), ZHANG XIANGXIN: XP002674775, Database accession no. CN1709394
• DATABASE TCM [Online] SIPO; 13 May 2005 (2005-05-13), QU WEIZHU: "HEALTH PROMOTING MATTRESS OF TEA", XP002674776, Database accession no. CN2805531

EP 2 275 113 B1

- **KOJI UMEKAWA ET AL.: 'Konenkishogai to Aromatherapy - Rose to Rosemary o Chushin ni' OBSTETRICS & GYNECOLOGY vol. 66, no. 10, 1999, pages 1350 - 1354**
- **KAZUHIKO SAWADA ET AL.: 'Mori no Kaori no Seiri Sayo' THE JAPANESE JOURNAL OF TASTE AND SMELL RESEARCH vol. 6, no. 3, 1999, pages 465 - 468, XP002937875**
- **HONGRATANAWORAKIT TAPANEE: 'Relaxing effect of rose oil on humans' NATURAL PRODUCT COMMUNICATIONS vol. 4, no. 2, February 2009, pages 291 - 296, XP001539146**

## Description

Technical Field

This invention relates to a pharmaceutical preparation or quasi-drug for use in improvement of skin circulation, for increasing a rate of skin blood circulation, for elevating of skin temperature or for prevention or improvement of shoulder stiffness or sensitivety to cold and use of a cosmetic preparation, a food or a drink for improvingg skin circulation, in elevation of skin temperature or for preventing or improving shoulder stiffness or sensitivity to cold.

Background Art

In the modern society, there has been an increase in the number of persons who complain of symptoms of sensitivity of the limbs to cold, numbness of the limbs, shoulder stiffness, swelling, and the like, due to various factors, such as lack of exercise, long-period labor in a specific posture at jobsites, air conditioning, and autonomic ataxia due to stress. It is thought that the symptoms described above are caused to occur primarily by a bad condition of blood circulation.

If the circulation of the blood becomes poor due to badness or interruption in blood circulation, or the like, the warm blood does not sufficiently circulate to the periphery. Also, supply of oxygen and nutrients from the blood becomes stagnant. Moreover, waste materials and water are accumulated.

Also, humans, who are homoiothermic animals, are provided with a body temperature regulating function for keeping the body temperature at a specific value. The autonomic nervous system and the endocrine system are regulated by a command given from a body temperature regulation center, which is located at the hypothalamus of the brain and has perceived a change (stimulus) of the ambient temperature, and the body temperature is thereby kept at the specific value. However, if balance of the autonomic nerve is lost due to a mental or physical stress, or the like, the body temperature regulating function does not work well, and the body cannot appropriately cope with the cold. Also, the sympathetic nerve becomes predominant, contraction of blood vessels and tension of the muscles are sustained, and the badness in blood circulation thus arises.

Various attempts have been made in order to improve the symptoms accompanying the bad condition of blood circulation described above. For example, in patent literature 1, it is described that a hyssop extract improves blood fluidity and blood circulation, increases skin surface temperature, and can improve sensitivity to cold and shoulder stiffness.

Also, in patent literature 2, it is described that a composition, which contains a cassis condensate and an amino acid or an organic acid, expands blood vessels, increases a peripheral blood circulation rate, and improves shoulder stiffness and sensitivity to cold.

Further, in patent literature 3, it is described that a smell of a chamomile extract improves blood circulation and increases peripheral skin temperature.

However, there are large individual differences in sensitivity and preference with respect to the aforesaid compounds and compositions having been reported heretofore, and sufficiently satisfactory results have not yet been obtained with the aforesaid compounds and compositions. Therefore, there is a strong demand for a novel substance, which can more efficiently and generally achieve improvement in skin blood circulation and increase in skin temperature.

In patent literature 4, effects of Ylang-Ylang oil has been examined on human physiological parameters and self-evaluation. Compared to odorless places Ylang-Ylang oil cause significant increases of subjective attentiveness and alterness. Correlational analysis reveal that the observed effects are mainly due to a subjective odor experience.

Patent literature 5 relates to a Chinese composition of plant origin, comprising Radix Angelicae Dahuricae, Radix Angelicae Pubescentis, Radix Ophiopogonis, Fructus Psoraleae, Radix Peucedani, Fructus Foeniculi, Herba Agrimoniae and Fructus Atriplicis Sibiricae, and said composition supports skin carrying and blood circulation and promotes the blood supply of skin affected part.

Patent literature 6 discloses a health promoting mattress of tea comprising a Chinese composition containing Flos Jasmini Sambac. Said mattress also exerts back stiffness inhibiting effects.

Patent literature 1:
Japanese Unexamined Patent Publication No. 2006-8575

Patent literature 2:
Japanese Unexamined Patent Publication No. 2004-262878

Patent literature 3:
Japanese Unexamined Patent Publication No. 2005-68069

Patent literature 4:
Hongratanaworakit T. et al., Medicinal and Aromatic Plants 2004, 26, 632-636

Patent literature 5:
Database TCM, Zhang Xiangxin: Database accession no. CN1709394

Patent literature 6:
Database TCM, Qu Weizhu: Database accession no. CN2805531

Disclosure of Invention

Problems To Be Solved by the Invention

In view of the above circumstances, the object of the present invention is to provide a novel composition for use in improvement of skin circulation, a novel composition for use in elevation of skin temperature, and a novel composition for use in prevention or improvement of shoulder stiffness or sensitivity to cold, which can safely and efficiently achieve increase in a rate of skin blood circulation and increase in skin temperature and can efficiently improve sensitivity to cold, shoulder stiffness, and the like.

Means for Solving the Problems

The above problems are solved the by subject-matter in accordance with the independent claims. Preferred embodiments result from the sub-claims. The inventors have found that each of an extract of Arabian jasmine (matsurika, jasmin sambac), which is a plant of the Jasminum genus in the Oleaceae family, 3-methyloctano-4-lactone, and bornyl acetate increases the rate of the skin blood circulation and increases the skin temperature. The present invention is based upon the findings described above.

Each of a pharmaceutical preparation or a quasi-drug for use in improvement of skin circulation, in elevation of skin temperature or in prevention or improvement of shoulder stiffness or sensitivity to cold and use of a cosmetic preparation, a food or a drink for improving skin circulation, for elevating skin temperature or for preventing or improving shoulder stiffness or sensitivity to cold in accordance with the present invention is characterized by containingat least one of the members selected from the group consisting of an Arabian jasmine (matsurika, jasmin sambac) extract and 3-methyloctano-4-lactone. The inventors have confirmed that each of the above-enumerated compounds has a sympathetic nerve suppressing effect. It is presumed that, in cases where the autonomic nerve is sedately adjusted to regulate such that the working of the parasympathetic nerve becomes relatively predominant over the working of the sympathetic nerve, the blood circulation is improved through expansion of blood vessels and relief of the tension of the muscles, and the skin temperature increases by virtue of the warm blood circulating to the periphery through the good blood circulation.

Each of a pharmaceutical preparation or a quasi-drug for use in prevention or improvement of shoulder stiffness or sensitivity to cold in elevation of skin temperature or in prevention or improvement of shoulder stiffness or sensitivity to cold and use of a cosmetic preparation, a food or a drink for improving skin circulation, for elevating skin temperature or for preventing or improving shoulder stiffness or sensitivity to cold in accordance with the present invention is characterized by containingat least one of the members selected from the group consisting of an Arabian jasmine (matsurika, jasmin sambac) extract and 3-methyloctano-4-lactone. In cases where the skin blood circulation is improved, and the skin temperature is increased, the warm blood circulates to the periphery, and metabolism is enhanced. Also, the supply of oxygen and nutrients from the blood and excretion of waste materials are enhanced, and the symptoms of the sensitivity to cold and the shoulder stiffness can be alleviated.

Effects of the Invention

Each of the skin circulation-improving agent comprised in the pharmaceutical preparation or quasi-drug for use in improvement of skin circulation, the skin temperature-elevating agent comprised in the cosmetic preparation used for use in elevation of skin temperature, and the like, in accordance with the present invention does not have a side effect, can increase the peripheral blood circulation rate or the skin temperature without being influenced by the sensitivities and the preferences of the individuals, and can safely and efficiently improve various symptoms due to a bad condition of blood circulation, such as the sensitivity to cold and the shoulder stiffness.

Best Mode for Carrying Out the Invention

Each of the skin circulation-improving agent comprised in the pharmaceutical preparation or quasi-drug for use in improvement of skin circulation, the skin temperature-elevating agent comprised in the pharmaceutical preparation or quasi-drug for use in elevation of skin temperature, the agent for preventing or improving shoulder stiffness, and the agent for preventing or improving sensitivity to cold comprised in pharmaceutical preparation or quasi-drug for use in prevention or improvement of shoulder stiffness or sensitivity to cold (hereinbelow referred to as the skin circulation-improving agent or the like) in accordance with the present invention contains at least one of the members selected from the group consisting of the Arabian jasmine (matsurika, jasmin sambac) extract, 3-methyloctano-4-lactone.

The term "skin circulation improving" as used herein means that the rate of the blood circulation through the skin blood vessels is increased. For example, the improvement in skin circulation can be evaluated by fitting a probe to a finger, a toe, or the like, and measuring a change in blood circulation rate by use of a laser Doppler flow-meter (ADVANCE LASER FLOW-METER ALF21), or the like.

Also, the term "skin temperature elevating" as used herein means that the skin temperature is increased. For example, the elevation of the skin temperature can be evaluated by measuring a change in skin temperature of a cheek, a finger, a toe, or the like, by use of an 8ch body temperature logger (supplied by Nikkiso-Therm Co., Ltd.), or the like.

Further, the term "preventing or improving" as used herein means that the occurrence of the symptoms is prevented, or that the symptoms are alleviated or relieved.

The Arabian jasmine (matsurika, jasmin sambac) is the plant of the Jasminum genus in the Oleaceae family. Whole grass, leaves, bark, branches, fruits, roots, or the like, of the Arabian jasmine can be used as such or after being pulverized. Particularly, flowers should preferably be used. The Arabian jasmine extract embraces in its scope an extract obtained from processing, wherein the extract part of the plant is subj ected as such to the extraction, or processing, wherein the extract part of the plant is subjected to cutting into appropriate sizes or pulverization after being dried and is then subjected to the extraction, and a more active fraction (ingredient) obtained by further subjecting the aforesaid extract to separation and purification. For the extraction, it is possible to employ a solvent extraction technique, wherein the extract part of the plant is impregnated with a solvent at the room temperature or in a heated state, or wherein the extraction is performed by use of an extraction appliance, such as a Soxhlet's extractor, an extraction technique using a distillation technique, such as steam distillation, a super critical extraction technique, wherein the extraction is performed with a carbonic acid gas being set in a super critical state, a press technique, wherein an extract is obtained through pressing, or the like. Particularly, the extract obtained from the solvent extraction can be used preferably.

As the extraction solvent used for the solvent extraction, a polar solvent or a non-polar solvent can be used, and a mixture thereof can also be used. Examples of the extraction solvents include water; alcohols, such as methanol, ethanol, propanol, and butanol; polyhydric alcohols, such as ethylene glycol, propylene glycol, and butylene glycol; ketones, such as acetone and methyl ethyl ketone; esters, such as methyl acetate and ethyl acetate; chain and cyclic ethers, such as tetrahydrofuran and diethyl ether; polyethers, such as polyethylene glycol; halogenated hydrocarbons, such as dichloromethane, chloroform, and carbon tetrachloride; hydrocarbons, such as hexane, cyclohexane, and petroleum ether; aromatic hydrocarbons, such as benzene and toluene; pyridines; super critical carbon dioxide; fats and oils; wax; and other kinds of oils. Each of the above-enumerated solvents can be used alone, or at least two of the above-enumerated solvents can be used in combination. It is also possible to iterate extraction processes by using different kinds of the solvents. Of the above-enumerated solvents, it is preferable to use ethanol, dipropylene glycol, propylene glycol, and butylene glycol. The extraction can be performed, for example, by using 1 part to 50 parts by mass of the solvent with respect to 1 part by mass of the plant, and carrying out dipping or heat reflux at a temperature falling within the range of 3°C to 100°C for a period of time falling within the range of several hours to several weeks.

The Arabian jasmine extract may further be subjected to the separation and the purification. Examples of means for the separation and the purification include activated carbon treatment, liquid-liquid distribution, column chromatography, liquid chromatography, gel filtration, and precision distillation.

Each of 3-methyloctano-4-lactone used in the present invention and bornyl acetate (disclosed herein but not in accordance with the invention) can be synthesized by use of a known synthetic technique. Alternatively, a commercially available product may be used.

In the present invention, one of the members selected from the group consisting of the Arabian jasmine extract and 3-methyloctano-4-lactone may be used alone. Alternatively, both of the members selected from the group described above may be used in combination. In so far as the skin circulation-improving agent comprised in the pharmaceutical preparation or quasi-drug for use in improvement of skin circulation or the like in accordance with the present invention contains at least one of the aforesaid substances as the active ingredient and can accomplish the effects of the present invention, the skin circulation-improving agent or the like can be used in an form, such as a liquid, a paste, a gel, or a solid. Also, the skin circulation-improving agent comprised in the composition for use in improvement of skin circulation or the like in accordance with the present invention can contain other arbitrary ingredients, such as carriers, diluents, and auxiliaries, within a range such that the effects of the present invention are not obstructed.

The skin circulation-improving agent or the like can be applied to use applications, which are pharmaceutical preparations, quasi-drugs, cosmetic preparations, foods, and drinks. Further described herein, since the active ingredient described above can exhibit the effects through vaporization, the skin circulation-improving agent or the like can also be applied to clothes, daily-use goods, and the like. The object, in which the skin circulation-improving agent or the like is contained, may be an arbitrary object, which can contain the skin circulation-improving agent or the like in a form such that the active ingredient described above can be vaporized and inhaled as vapor. In accordance with the kind of the object, the skin circulation-improving agent or the like may further contain arbitrary constituent elements, which are ordinarily contained in the object, besides the active ingredient described above. Furthermore, other arbitrary active drugs and the skin circulation-improving agent or the like may be used together with each other.

The product forms containing the skin circulation-improving agent or the like include cosmetic preparations, pharmaceutical preparations, quasi-drugs, foods, and drinks. Examples of the preparation forms include liquids, powders, granules, aerosols, solids, and gels.

Examples of the cosmetic preparations, which are one of particularly preferable embodiments, include perfumes, eau de toilette, eau de Cologne, creams, milky lotions, skin lotions, foundations, face powders, lipsticks, soaps, shampoos and rinses, body shampoos, body rinses, body powders, and bath preparations.

Also, the skin circulation-improving agent or the like can be contained in arbitrary daily-use goods, such as aromatic products, deodorants, aroma candles, incense, writing materials, purses, bags, and shoes; and arbitrary clothes, such as underwear, dresses, hats and caps, stockings, and socks. The skin circulation-improving agent or the like may be added to the materials for the daily-use goods and the clothes described above or the products of the daily-use goods and the clothes described above.

The skin circulation-improving agent comprised in the composition for use in improvement of skin circulation or the like in accordance with the present invention can be used in various embodiments exemplified above. In so far as the effects of the present invention can be accomplished, the skin circulation-improving agent comprised in the composition for use in improvement of skin circulation or the like in accordance with the present invention can be used in arbitrary embodiments.

Examples

The present invention will further be illustrated by the following examples. As the Arabian jasmine solvent extract, that commercially available from Robertet was used. As 3-methyloctano-4-lactone, the compound commercially available from Soda Aromatic Co., Ltd. was used. Also, as bornyl acetate (not according to the invention), the compound commercially available from Takasago International Corporation was used.

Test Example 1: Study of effect on skin temperature

The skin temperature of fingers was measured by use of 8ch Body Temperature Logger (supplied by Nikkiso-Therm Co., Ltd.), and a change in skin temperature due to smelling of a test substance was investigated. The effect on the peripheral skin temperature was thus studied. A solvent extract of a common jasmine (Jasminum officinale) belonging to the Jasminum genus in the Oleaceae family was used as a control, and the evaluation was made in the same manner.

A woman in her twenties was employed as a subject, and the test was made in a constant temperature and humidity room at a temperature of 25°C and a humidity of 50%.

The subject was seated on an easy chair and allowed to take a rest for 30 minutes. Thereafter, body temperature sensors were fitted to inner surface sides of third fingers of both hands of the subject and to two sites of the chest of the subject, and a change in skin temperature at each site with the passage of time was recorded. Since it had been found that little change arose in skin temperature at the chest, the skin temperatures at the chest were employed as reference values. The change in peripheral skin temperature was evaluated by use of a value (T value) obtained by subtracting a mean value of the skin temperatures at the two sites of the chest from the mean value of the skin temperatures at the two fingertip sites.

One sequence of measurement tests was composed of (A) a rest for three minutes, (B) attaching of an odorless cotton piece under the nose for three minutes, (C) a rest for three minutes, (D) attaching of a test sample-impregnated cotton piece under the nose for three minutes, (E) a rest for three minutes, (F) attaching of an odorless cotton piece under the nose for three minutes, and (G) a rest for three minutes. The skin temperature was measured successively during the one sequence, and the T values were calculated.

The T values obtained at the times of (B) and (F), at each of which the odorless cotton piece was attached, were taken as the controls and were compared with the T value obtained at the time of (D), at which the vapor of the test sample was inhaled. The effect of each substance on the skin temperature was thus investigated. Specifically, the formula shown below was utilized.

$$\text{Skin temperature change (°C)}$$

$$= \text{T value (D) - (mean value of T value (B) and T value (F))}$$

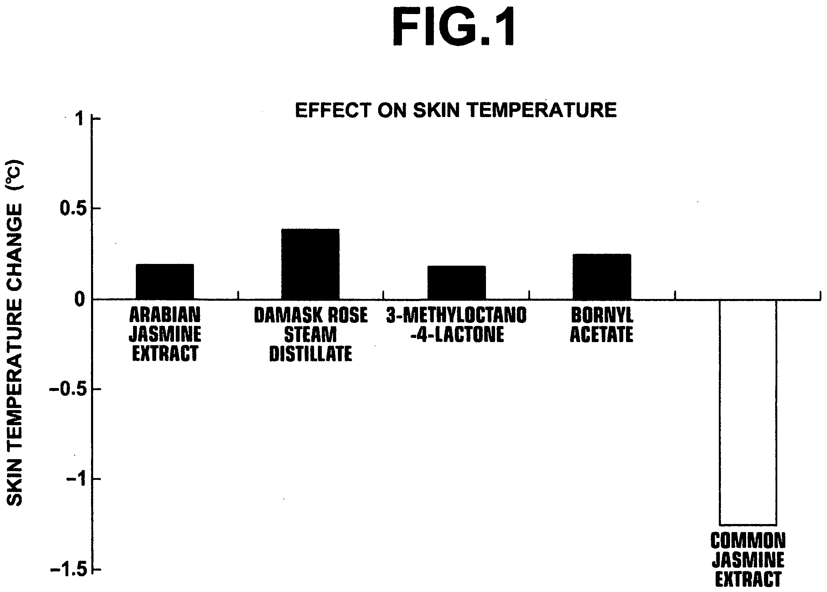

The results are shown in Figure 1. It was indicated that, in cases where the subject was allowed to take the smell of the Arabian jasmine extract, 3-methyloctano-4-lactone, or bornyl acetate with the nose, the skin temperatures of the fingers of the subject were increased, and that each of these substances thus had the skin temperature increasing effect.

Also, though it is not illustrated by data, in cases where the subject was allowed to take the smell of the Arabian jasmine extract, 3-methyloctano-4-lactone, or bornyl acetate with the nose, the skin temperatures of the toes and the cheeks of the subject could be increased.

Test Example 2: Study of effect on skin blood circulation

The skin blood circulation rate of fingers was measured by use of the laser Doppler flow-meter (ADVANCE LASER FLOW-METER ALF21), and the effect of the Arabian jasmine extract on the skin blood circulation was evaluated.

Six persons (men and women) were employed as subjects, and the test was made in a constant temperature and humidity room having been adjusted at a temperature of 22°C and a humidity of 45%. Each of the subjects was seated on an easy chair and allowed to take a rest for 30 minutes. Thereafter, a probe of the flow-meter was fitted to the inner surface side of the third finger of the right hand of the subject, and a change in blood circulation rate with the passage of time was recorded. One sequence of measurement tests was composed of (A) a rest for three minutes, (B) attaching of an odorless cotton piece under the nose for three minutes, (C) a rest for three minutes, (D) attaching of a test sample-impregnated cotton piece under the nose for three minutes, (E) a rest for three minutes, (F) attaching of an odorless cotton piece under the nose for three minutes, and (G) a rest for three minutes. The blood circulation rate was measured successively during the one sequence.

The blood circulation rates obtained at the times of (B) and (F), at each of which the odorless cotton piece was attached, were taken as the controls and were compared with the blood circulation rate obtained at the time of (D), at which the vapor of the test sample was inhaled. The effect of each substance on the skin blood circulation rate was thus investigated. Specifically, the formula shown below was utilized.

$$\text{Blood circulation rate change (ml/min/100g) = blood circulation rate (D) - (mean value of}$$

$$\text{blood circulation rate (B) and blood circulation rate (F))}$$

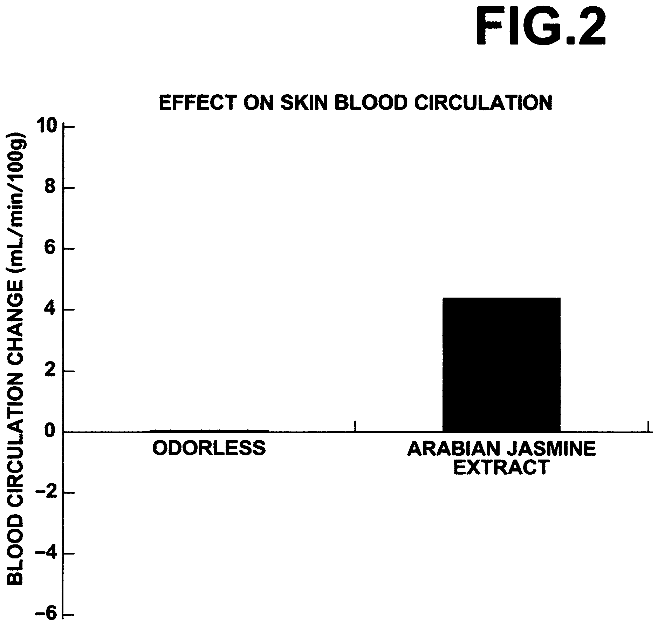

The results are shown in Figure 2. It was indicated that, in cases where each of the subjects was allowed to take the smell of the Arabian jasmine extract with the nose, the skin blood circulation rate was increased, and that the Arabian jasmine extract thus had the skin blood circulation improving effect.

Test Example 3: Study of effects on symptoms of sensitivity to cold and shoulder stiffness

As one of causes of the sensitivity to cold, the shoulder stiffness, and the like, the decreasing in blood circulation is considered. Therefore, the improvement effects of the Arabian jasmine extract on the symptoms of the sensitivity to cold and the shoulder stiffness were investigated.

Four persons (men and women) aware of the sensitivity to cold were employed as the subjects, and the test was made in a constant temperature and humidity room having been adjusted at a temperature of 22°C and a humidity of 45%. Each of the subjects was seated on an easy chair and allowed to take a rest for 30 minutes. Thereafter, an odorless cotton piece was attached under the nose of the subject. After a period of time of three minutes had elapsed, each of the extents of the sensitivity to cold and the shoulder stiffness was evaluated subjectively on a five-point scale. Thereafter, a cotton piece impregnated with the Arabian jasmine extract was attached under the nose, and the vapor of the extract was inhaled with natural breathing for three minutes. Thereafter, each of the extents of the sensitivity to cold and the shoulder stiffness was again evaluated subjectively on the five-point scale.

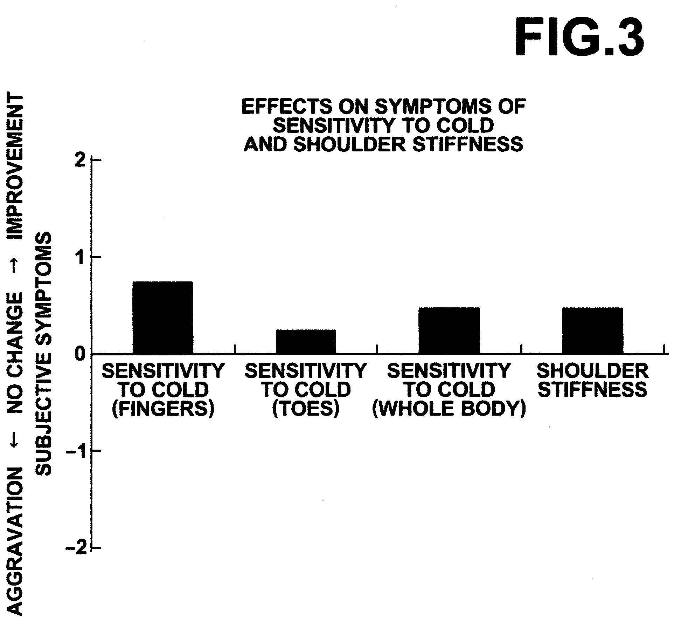

The results are shown in Figure 3. It was indicated that, in cases where each of the subjects was allowed to take the smell of the Arabian jasmine extract with the nose, the results of the warming of the hands, feet, and the entire body were obtained as an actual feeling and that, in such cases, the symptoms of the shoulder stiffness were improved.

Application examples of typical product forms, such as various compositions, daily-use goods, and clothes, containing the skin circulation-improving agent, the skin temperature-elevating agent, the agent for preventing or improving shoulder stiffness, or the agent for preventing or improving sensitivity to cold will be described hereinbelow. In each of the application examples, the containing quantity is expressed in terms of % by mass with respect to the total quantity of the product. As the skin circulation-improving agent comprised in the composition for use in the improvement of skin circulaton or the like in accordance with the present invention, one of the members selected from the group consisting of the Arabian jasmine extract, 3-methyloctano-4-lactone, and bornyl acetate (not according to the invention) was contained alone, or at least two of the members selected from the group described above were contained in combination.

Application Example 1

Skin lotion

(1) Glycerol 2.0

(2) Dipropylene glycol 2.0

(3) PEG-60 hydrogenated castor oil 0.3

(4) Trimethylglycine 0.1

(5) Preservative Proper quantity

(6) Chelating agent Proper quantity

(7) Dye Proper quantity

(8) Skin circulation-improving agent
(Arabian jasmine (matsurika, jasmin sambac) extract) 0.05

(9) Purified water Balance

Application Example 2 (not according to the invention)

Skin lotion

(1) Alcohol 30.0
(2) Butylene glycol 4.0
(3) Glycerol 2.0
(4) PPG-13 decyltetradeth-24 0.3
(5) Octylmethoxy cinnamate 0.1
(6) Menthol 0.2
(7) Tocopheryl acetate 0.1
(8) Chelating agent Proper quantity
(9) Dye Proper quantity
(10) Skin circulation-improving agent (bornyl acetate) 0.01
(11) Purified water Balance

Application Example 3

Milky lotion

(1) Stearic acid 2.0

(2) Cetyl alcohol 1.5
(3) Vaseline 4.0
(4) Squalane 5.0
(5) Glycerol tri-2-ethylhexanoic acid ester 2.0
(6) Sorbitan monooleic acid ester 2.0
(7) Dipropylene glycol 5.0
(8) PEG1500 0.3
(9) Triethanolamine 0.1
(10) Preservative Proper quantity
(11) Skin circulation-improving agent (3-methyloctano-4-lactone) 0.2
(12) Purified water Balance

Application Example 4 (not according to the invention)

Milky lotion

(1) Ethyl alcohol 10.0
(2) Cyclomethicone 0.1
(3) Butylene glycol 5.0
(4) Dimethicone 3.0
(5) Glycene 0.1
(6) Menthol 1.0
(7) Trimethylsiloxysilicic acid 0.1
(8) Caffeine 1.0
(9) Trimethylglycine 1.0
(10) Xanthan gum 0.001
(11) Hydroxyethyl cellulose 0.1
(12) Soybean fermentation extract 1.0
(13) Lauryl betaine 0.5
(14) Carbomer 0.2
(15) Chelating agent Proper quantity
(16) Paraben Proper quantity
(17) Benzoic acid Proper quantity
(18) Skin temperature-elevating agent (bornyl acetate) 0.1
(19) Iron oxide Proper quantity
(20) Potassium hydroxide 0.05
(21) Dicalcium glycyrrhizinate 0.01
(22) Pyridoxine hydrochloride 0.01
(23) Ascorbic acid glucoside 0.01
(24) Arbutin 3.0
(25) Extract of Saxifraga stolonifera Meerb. 0.1
(26) Water Balance

Application Example 7 (not according to the invention)

Milky lotion

(1) Ethanol 2.0
(2) Cyclomethicone 10.0
(3) Glycerol 5.0
(4) Dibutylene glycol 1.0
(5) Dimethicone 1.0
(6) Corn starch 4.0
(7) Mineral oil 2.0

(8) Trimethylsiloxysilicic acid 5.0
(9) Polyethylene glycol 3.0
(10) Menthyl lactate 0.1
(11) PEG-60 hydrogenated castor oil 1.0
(12) Aminopropyl dimethicone 1.0
(13) Xanthan gum 0.01
(14) Tocopheryl acetate 0.01
(15) Caffeine 0.1
(16) Sodium hyaluronate 0.1
(17) Soybean fermentation extract 0.01
(18) Hamamelis extract 0.01
(19) Extract of Houttuynia cordata 0.01
(20) Carbomer 0.3
(21) Acrylic acid-alkyl matacrylate copolymer 0.2
(22) EDTA Proper quantity
(23) Preservative Proper quantity
(24) Skin temperature-elevating agent (bornyl acetate) 0.3
(25) Pigment Proper quantity
(26) Potassium hydroxide 0.15
(27) Aminomethylpropanol 0.05
(28) Water Balance

Application Example 8

Milky lotion

(1) Ethanol 15.0
(2) Cyclomethicone 6.0
(3) Butylene glycol 0.5
(4) Dimethicone 1.0
(5) Glycerol 1.0
(6) Polyethylene glycol 1.0
(7) Menthyl lactate 1.0
(8) Menthol 0.1
(9) Trimethylsiloxysilicic acid 1.0
(10) Caffeine 0.5
(11) Trimethyleneglycine 0.1
(12) Xanthan gum 0.1
(13) Hydroxyethyl cellulose 0.1
(14) Soybean fermentation extract 0.01
(15) Tocopheryl acetate 0.05
(16) Lauryl betaine 0.01
(17) Brown algae extract 0.01
(18) Extract of Houttuynia cordata 0.01
(19) Red algae extract 0.01
(20) Green algae extract 0.01
(21) Cellulose powder 1.0
(22) PEG-60 glyceryl isostearate 1.0
(23) Isostearic acid 1.0
(24) Carbomer 0.1
(25) Acrylic acid-alkyl methacrylate copolymer 0.1
(26) EDTA 0.1
(27) Sodium metaphosphate 0.1
(28) Phenoxy ethanol 0.2
(29) Paraben 0.2
(30) Skin temperature-elevating agent (3-methyloctano-4-lactone) 0.20

(31) Skin temperature-elevating agent (bornyl acetate) 0.15
(32) Iron oxide (red) 0.02
(33) Menthyl glyceryl ether 0.01
(34) Water Balance

Application Example 10

Cream

(1) Glycerol 3.0

(2) Dipropylene glycol 7.0

(3) Polyethylene glycol 3.0

(4) Glyceryl stearate 3.0

(5) Glyceryl isostearate 2.0

(6) Stearyl alcohol 2.0

(7) Behenyl alcohol 2.0

(8) Liquid paraffin 7.0

(9) Cyclomethicone 3.0

(10) Dimethicone 1.0

(11) Octylmethoxy cinnamate 0.1

(12) Sodium hyaluronate 0.05

(13) Preservative Proper quantity

(14) Antioxidant Proper quantity

(15) Skin circulation-improving agent (Arabian jasmine (matsurika, jasmin sambac) extract) 0.3

(16) Skin circulation-improving agent (3-methyloctano-4-lactone) 0.1

(17) Purified water Balance

(18) Chelating agent Proper quantity

(19) Pigment Proper quantity

Application Example 12

Aerosol

(1) Glycerol 2.0

(2) Dipropylene glycol 2.0

(3) PEG-60 hydrogenated castor oil 0.3

(4) HPPCD 1.0

(5) Preservative Proper quantity

(6) Chelating agent Proper quantity

(7) Dye Proper quantity

(8) Skin circulation-improving agent (Arabian jasmine (matsurika, jasmin sambac) extract) 0.2

(9) Purified water Proper quantity

(10) LPG Balance

Application Example 13 (not according to the invention)

Aerosol

(1) Alcohol 15.0

(2) Butylene glycol 2.0

(3) Glycerol 1.0

(4) PPG-13 decyltetradeth-24 0.1

(5) Silver-carrying zeolite 1.0

(6) Chelating agent Proper quantity

(7) Dye Proper quantity

(8) Skin circulation-improving agent (bornyl acetate) 0.15

(9) Purified water Balance

(10) LPG 40.0

Application Example 15 (not according to the invention)

Shampoo

(1) Lauryl polyexyethylene (3) sulfuric acid ester sodium salt
(30% aqueous solution) 30.0

(2) Lauryl sulfuric acid ester sodium salt (30% aqueous solution) 10.0

(3) Coconut oil fatty acid diethanolamide 4.0

(4) Glycerol 1.0

(5) Preservative Proper quantity

(6) Skin circulation-improving agent (bornyl acetate) 0.5

(7) Colorant Proper quantity

(8) Sequestering agent, pH adjustor Proper quantity

(9) Purified water Balance

Application Example 18

Fragrance

(1) Alcohol 75.0

(2) Purified water Balance

(3) Dipropylene glycol 5.0

(4) Skin temperature-elevating agent (3-methyloctano-4-lactone) 10.0

(5) Antioxidant 8.0

(6) Colorant Proper quantity

(7) UV-absorber Proper quantity

The term "fragrance" as used herein means the solution of the essential oil dissolved in an alcohol (e.g., ethyl alcohol) or an aqueous alcohol. The fragrance contains the essential oil in a containing quantity falling within the range of 1% to 99% by mass. The containing ratio of water to the alcohol falls within the range between 50:50 and 0:100. The fragrance can contain solubilizers, softening agents, humectants, thickening agents, bacteriostatic agents, or other materials which are ordinarily used in cosmetic preparations.

Application Example 19

Room fragrance

(1) Alcohol 80.0

(2) Purified water Balance

(3) Antioxidant 5.0

(4) Skin circulation-improving agent (Arabian jasmine (matsurika, jasmin sambac) extract) 2.0

(5) Skin circulation-improving agent (3-methyloctano-4-lactone) 1.0

(6) 3-Methyl-3-methoxy butanol 5.0

(7) Dibenzylidene sorbitol 5.0

Application Example 20 (not according to the invention)

Incense

(1) Machilus thunbergii powder 75.5

(2) Sodium benzoate 15.5

(3) Skin circulation-improving agent (bornyl acetate) 5.0

(4) Eucalyptus oil 1.0

(5) Fennel oil 1.0

(6) Purified water Balance

Application Example 23 (not according to the invention)

Massage cream

(1) Solid paraffin 5.0
(2) Beeswax 10.0
(3) Vaseline 15.0
(4) Liquid paraffin 41.0
(5) 1,3-Butylene glycol 4.0
(6) Glycerol monostearate 2.0
(7) POE (20) sorbitan monolauric acid ester 2.0
(8) Borax 0.2
(9) Caffeine 2.0
(10) Preservative Proper quantity
(11) Antioxidant Proper quantity
(12) Skin circulation-improving agent (bornyl acetate) 1.0
(13) Purified water Balance

Application Example 26

Tablet (chewable type)

(1) Inositol 11.0
(2) Maltitol 21.0
(3) Sucrose 0.5
(4) Salmon milt extract (DNA Na) 0.1
(5) Yeast extract 0.1
(6) Skin circulation-improving agent
(3-methyloctano-4-lactone) 0.1
(7) Flavoring 5.0
(8) Excipient Balance

Application Example 27

Tablet

(1) Lubricant (sucrose fatty acid ester, or the like) 1.0

(2) Aqueous gum arabic solution (5%) 2.0

(3) Acid taste agent 1.0

(4) Colorant Proper quantity

(5) Skin temperature-elevating agent (Arabian jasmine (matsurika, jasmin sambac) extract) 0.1

(6) Glucide (sugar powder, sorbitol, or the like) Balance

Application Example 28 (not according to the invention)

Candy

(1) Sugar 50.0

(2) Thick malt syrup 47.95

(3) Organic acid 2.0

(4) Skin temperature-elevating agent (bornyl acetate) 0.05

By use tests having been made in typical embodiments of the corresponding product forms, it was confirmed that each of the various kinds of the compositions, the daily-use goods, the clothes, and the like, obtained in the application examples described above could improve the shoulder stiffness or the sensitivity to cold.

Brief Description of Drawings

Figure 1 is a graph showing an effect on skin temperature,
Figure 2 is a graph showing an effect on skin blood circulation, and
Figure 3 is a graph showing effects on symptoms of sensitivity to cold and shoulder stiffness.

**Claims**

**1.** A pharmaceutical preparation or a quasi-drug for use in improvement of skin circulation, containing at least one of the members selected from the group consisting of an Arabian jasmine (matsurika, jasmin sambac) extract and 3-methyloctano-4-lactone.

**2.** A pharmaceutical preparation or a quasi-drug for use in elevation of skin temperature, containing at least one of the members selected from the group consisting of an Arabian jasmine (matsurika, jasmin sambac) extract and 3-methyloctano-4-lactone.

**3.** A pharmaceutical preparation or a quasi-drug for use in prevention or improvement of shoulder stiffness or sensitivity to cold, containing at least one of the members selected from the group consisting of an Arabian jasmine (matsurika, jasmin sambac) extract and 3-methyloctano-4-lactone.

4. Use of a cosmetic preparation, a food or a drink containing at least one of the members selected from the group consisting of an Arabian jasmine (matsurika, jasmin sambac) extract, and 3-methyloctano-4-lactone for improving skin circulation.

5. Use of a cosmetic preparation, a food or a drink containing at least one of the members selected from the group consisting of an Arabian jasmine (matsurika, jasmin sambac) extract, and 3-methyloctano-4-lactone for elevating skin temperature.

6. Use of a cosmetic preparation, a food or a drink containing at least one of the members selected from the group consisting of an Arabian jasmine (matsurika, jasmin sambac) extract, and 3-methyloctano-4-lactone for preventing or improving shoulder stiffness or sensitivity to cold.

**Patentansprüche**

1. Pharmazeutische Zubereitung oder Quasi-Arzneimittel zur Verwendung zur Verbesserung der Hautzirkulation, umfassend mindestens einen Bestandteil, ausgewählt aus der Gruppe, welche aus arabischem Jasmin (Matsurika, Jasmin sambac)-Extrakt und 3-Methyloctan-4-lacton besteht.

2. Pharmazeutische Zubereitung oder Quasi-Arzneimittel zur Verwendung zur Erhöhung der Hauttemperatur, umfassend mindestens einen Bestandteil, ausgewählt aus der Gruppe, welche aus arabischem Jasmin (Matsurika, Jasmin sambac)-Extrakt und 3-Methyloctan-4-lacton besteht.

3. Pharmazeutische Zubereitung oder Quasi-Arzneimittel zur Verwendung zur Verhinderung oder Verbesserung von Verspannungen der Schulter oder Kälteempfindlichkeit, umfassend mindestens einen Bestandteil, ausgewählt aus der Gruppe, welche aus arabischem Jasmin (Matsurika, Jasmin sambac)-Extrakt und 3-Methyloctan-4-lacton besteht.

4. Verwendung einer kosmetischen Zubereitung, eines Nahrungsmittels oder eines Getränks, umfassend mindestens einen Bestandteil, ausgewählt aus der Gruppe, welche aus arabischem Jasmin (Matsurika, Jasmin sambac)-Extrakt und 3-Methyloctan-4-lacton besteht, zur Verbesserung der Hautzirkulation.

5. Verwendung einer kosmetischen Zubereitung, eines Nahrungsmittels oder eines Getränks, umfassend mindestens einen Bestandteil, ausgewählt aus der Gruppe, welche aus arabischem Jasmin (Matsurika, Jasmin sambac)-Extrakt und 3-Methyloctan-4-lacton besteht, zur Erhöhung der Hauttemperatur.

6. Verwendung einer kosmetischen Zubereitung, eines Nahrungsmittels oder eines Getränks, umfassend mindestens einen Bestandteil, ausgewählt aus der Gruppe, welche aus arabischem Jasmin (Matsurika, Jasmin sambac)-Extrakt und 3-Methyloctan-4-lacton besteht, zur Verhinderung oder Verbesserung von Verspannungen der Schulter oder Kälteempfindlichkeit.

**Revendications**

1. Préparation pharmaceutique ou « quasi-drug » pour une utilisation dans l'amélioration de la circulation cutanée, qui contient au moins un des membres sélectionnés dans le groupe consistant en un extrait de jasmin d'Arabie (matsurika, Jasminum sambac) et la 3 méthyloctano-4-lactone.

2. Préparation pharmaceutique ou « quasi-drug » pour une utilisation dans l'augmentation de la température de la peau, qui contient au moins un des membres sélectionnés dans le groupe consistant en un extrait de jasmin d'Arabie (matsurika, Jasminum sambac) et la 3 méthyloctano-4-lactone.

3. Préparation pharmaceutique ou « quasi-drug » pour une utilisation dans la prévention ou l'amélioration d'une raideur de l'épaule ou de la sensibilité au froid, qui contient au moins un des membres sélectionnés dans le groupe consistant en un extrait de jasmin d'Arabie (matsurika, Jasminum sambac) et la 3 méthyloctano-4-lactone.

4. Utilisation d'une préparation cosmétique, d'une denrée alimentaire ou d'une boisson qui contient au moins un des membres sélectionnés dans le groupe consistant en un extrait de jasmin d'Arabie (matsurika, Jasminum sambac) et la 3 méthyloctano-4-lactone pour améliorer la circulation cutanée.

5. Utilisation d'une préparation cosmétique, d'une denrée alimentaire ou d'une boisson qui contient au moins un des membres sélectionnés dans le groupe consistant en un extrait de jasmin d'Arabie (matsurika, Jasminum sambac) et la 3 méthyloctano-4-lactone pour augmenter la température de la peau.

6. Utilisation d'une préparation cosmétique, d'une denrée alimentaire ou d'une boisson qui contient au moins un des membres sélectionnés dans le groupe consistant en un extrait de jasmin d'Arabie (matsurika, Jasminum sambac) et la 3 méthyloctano-4-lactone pour prévenir ou améliorer une raideur de l'épaule ou la sensibilité au froid.

# FIG.1

EFFECT ON SKIN TEMPERATURE

EP 2 275 113 B1

# FIG.2

EFFECT ON SKIN BLOOD CIRCULATION

# FIG.3

EFFECTS ON SYMPTOMS OF
SENSITIVITY TO COLD
AND SHOULDER STIFFNESS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006008575 A
- JP 2004262878 A
- JP 2005068069 A

**Non-patent literature cited in the description**

- **HONGRATANAWORAKIT T. et al.** *Medicinal and Aromatic Plants,* 2004, vol. 26, 632-636
- Database. CN1709394
- Database. CN2805531